# EUROPÄISCHE PATENTSCHRIFT

(11) **EP 1 023 272 B1**
(45) Veröffentlichungstag und Bekanntmachung des Hinweises auf die Patenterteilung: **16.07.2003**
(21) Anmeldenummer: 98955418.3
(22) Anmeldetag: 08.10.1998
(51) Int. Cl.: C07D 249/12, C07C 281/04

(54) **VERFAHREN ZUR HERSTELLUNG VON ALKOXYTRIAZOLINONEN**
PROCESS FOR PREPARING ALKOXYTRIAZOLINONES
PROCEDE DE PRODUCTION D'ALCOXYTRIAZOLINONES

(30) Priorität: 08.10.1997 CH 235497
(43) Veröffentlichungstag der Anmeldung: 02.08.2000
(73) Patentinhaber: Lonza AG, 4052 Basel (CH)
(72) Erfinder: VEITH, Ulrich, CH-3930 Visp (CH)
(74) Vertreter: Winter, Brandl & Partner
(86) Internationale Anmeldenummer: EP9806397
(87) Internationale Veröffentlichungsnummer: WO99018089

(56) Entgegenhaltungen:
- EP-A- 0 703 224
- EP-A- 0 703 225
- EP-A- 0 726 258
- DE-A- 4 433 967
- MACKAY D. ET AL.: "The 1:1 adducts of 2,5-dimethyl-3,4-diphenylcyclopentadienone and dimethylazodicarboxylate: a fourth isomer" CANADIAN JOURNAL OF CHEMISTRY., Bd. 61, Nr. 6, 1983, Seiten 1213-1217, XP002090338 OTTAWA CA

## Beschreibung

Die vorliegende Erfindung betrifft ein Verfahren zur Herstellung von Alkoxytriazolinonen der allgemeinen Formel 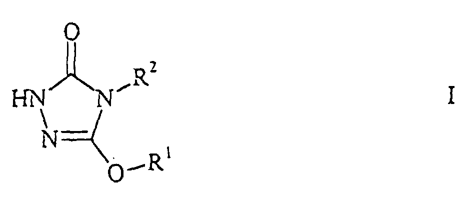 sowie einen neuen Hydrazincarbonsäureester der allgemeinen Formel 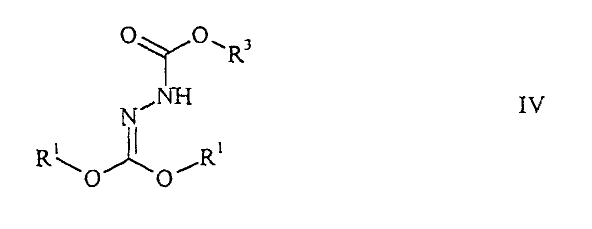 der als Zwischenprodukt bei der erfindungsgemässen Herstellung der Alkoxytriazolinone dient. Alkoxytriazolinone sind wichtige Zwischenprodukte für die Herstellung von agrochemischen Wirkstoffen.

R¹, R² und R³ haben unabhängig voneinander die Bedeutung einer gegebenenfalls substituierten geradkettigen oder verzweigten C₁₋₆-Alkylgruppe. Namentlich erwähnt seien Methyl, Ethyl, Propyl, Isopropyl, Butyl, Isobutyl, tert-Butyl, Pentyl und seine Isomeren sowie Hexyl und seine Isomeren. R¹ und R² können ausserdem unabhängig voneinander gegebenenfalls substituiertes Aryl, Arylalkyl, oder Cycloalkyl bedeuten. Aryl hat vorzugsweise die Bedeutung Phenyl. Arylalkyl bedeutet vorzugsweise Phenylalkyl, beispielsweise Phenyl-C₁₋₆-Alkyl, und besonders bevorzugt Benzyl. Cycloalkyl bedeutet insbesondere C₃₋₆-Cycloalkyl, vorzugsweise Cyclohexyl. Substituenten der Alkylgruppen, der Aromaten der Aryifunktion, oder der Cycloalkylgruppen sind Halogen, Amino, Alkylamino, Diaikylamino, Alkoxy oder Hydroxy, wobei Alkyl wie oben vorzugsweise C₁₋₆-Alkyl und Alkoxy bevorzugt C₁₋₆₋Alkoxy bedeutet. Unter Halogen ist hier und im folgenden Fluor, Chlor, Brom oder Jod zu verstehen. Bevorzugt hat R¹ die Bedeutung von Methyl, Propyl und Phenyl, besonders bevorzugt sind Methyl und Propyl. R² hat bevorzugt die Bedeutung von Methyl, Benzyl und Cyclohexyl. R³ hat bevorzugt die Bedeutung von Methyl.

Hydrazincarbonsäureester und Verfahren zu ihrer Herstellung werden in der EP-A-0 726 258 offenbart.

Die DE-A-44 33 967 und die EP-A-0 703 225 offenbaren Verfahren zur Herstellung von Alkoxytriazolinonen, worin zunächst ein Iminokohlensäurediester mit einem Carbazinsäureester zu einem Semicarbazid umgesetzt wird, das dann durch Cyclisierung und gegebenenfalls Reaktion mit einem Alkylierungsmittel in das gewünschte Alkoxytriazolinon überführt wird. Eine ähnliche Umsetzung wird in der EP-A-0 703 224 beschrieben, wobei hier als Ausgangsmaterial ein Iminothiokohlensäurediester eingesetzt wird.

Die Aufgabe der Erfindung war, ein ökonomisches, grosstechnisch gangbares Verfahren zur Herstellung von Alkoxytriazolinonen zur Verfügung zu stellen.
Diese Aufgabe wird durch das Verfahren nach Patentanspruch 1 sowie durch das neue Zwischenprodukt nach Patentanspruch 10 gelöst. Erfindungsgemäss wird in einer ersten Stufe ein Iminokohlensäurediester der allgemeinen Formel 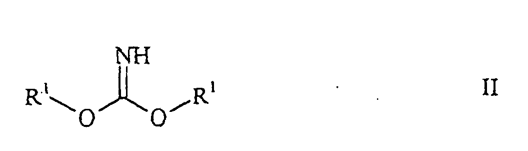 worin R¹ die genannte Bedeutung hat, in Gegenwart einer Mineralsäure und gelöst in Wasser oder in einem Gemisch aus Wasser mit einem mit Wasser mischbaren polaren organischen Lösungsmittel mit einem Carbazinsäureester der allgemeinen Formel 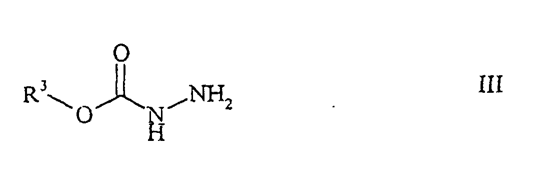 worin R³ die genannte Bedeutung hat, zu einem Hydrazincarbonsäureester der allgemeinen Formel 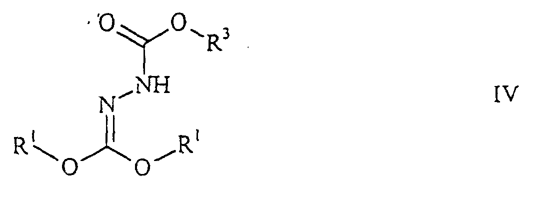 worin R¹ und R³ die genannte Bedeutung haben, umgesetzt.

Iminokohlensäurediester der allgemeinen Formel II können gemäss EP-A 0 523 619 oder gemäss Hantzsch (Chem. Ber., 1895, 28, 2470 - 2471) hergestellt werden. Carbazinsäureester sind gängige organische Synthesechemikalien.

Als Mineralsäure kann Salzsäure, Phosphorsäure oder Schwefelsäure eingesetzt werden. Besonders geeignet ist Salzsäure.

Mit Wasser mischbare polare organische Lösungsmittel sind zweckmässig geradkettige oder verzweigte Alkohole mit 1 bis 6 C-Atomen. Namentlich erwähnt seien Methanol, Ethanol, Propanol, Isopropanol, Butanol, Isobutanol oder tert-Butanol, Pentanol und seine Isomeren sowie Hexanol und seine Isomeren.

Die Umsetzung in der ersten Stufe erfolgt zweckmässig bei einer Temperatur von -5 bis 40 °C, vorteilhaft bei 0 bis 20 °C. Der pH liegt zweckmässig in einem Bereich von 3 bis 10, vorteilhaft in einem Bereich von 5 bis 8.
Nach einer üblichen Umsetzungszeit von 30 min bis 2 Stunden kann die bisher noch nicht in der Literatur beschriebene Verbindung der allgemeinen Formel IV durch übliche Methoden wie beispielsweise Extraktion isoliert werden oder direkt, ohne Isolation, für die zweite Stufe eingesetzt werden. Vorzugsweise wird das Zwischenprodukt (Formel IV) isoliert.

In einer zweiten Stufe wird der erfindungsgemässe Hydrazincarbonsäureester (Formel IV) mit einem Amin der allgemeinen Formel

R²―NH₂ V

worin R² die genannte Bedeutung hat, in Gegenwart von Wasser, einem polaren organischen Lösungsmittel oder Mischungen davon zum Endprodukt der Formel I umgesetzt. Bevorzugt erfolgt die Umsetzung in Gegenwart eines polaren organischen Lösungsmitteis.

Als polare organische Lösungsmittel werden zweckmässig geradkettige oder verzweigte Alkohole mit 1 bis 6 C-Atomen eingesetzt. Namentlich erwähnt seien Methanol, Ethanol, Propanol, Isopropanol, Butanol, Isobutanol oder tert-Butanol, Pentanol und seine Isomeren sowie Hexanol und seine Isomeren Besonders bevorzugt ist Methanol.

Die Umsetzung in der zweiten Stufe erfolgt zweckmässig bei einer Temperatur von 10 bis 200 °C, vorteilhaft bei 20 bis 100 °C.

Zweckmässig erfolgt die Umsetzung in der zweiten Stufe über ein Zwischenprodukt der allgemeinen Formel 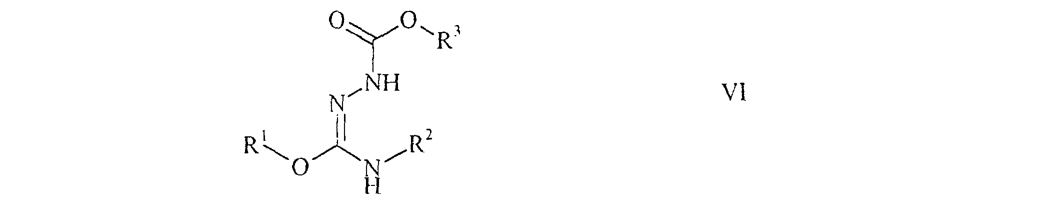 worin R¹, R² und R³ die genannte Bedeutung haben, welches gegebenenfalls isoliert werden kann.
Die Umsetzung in der zweiten Stufe wird zweckmässig mit einem Überschuss an Amin der allgemeinen Formel V bezogen auf den Hydrazincarbonsäureester der allgemeinen Formel IV durchgeführt. Bevorzugt wird das Amin der allgemeinen Formel V im Verhältnis 1,1: 1 bis 30 : 1, besonders bevorzugt im Verhältnis 2 : 1 bis 20 : 1, bezogen auf den Hydrazincarbonsäureester der allgemeinen Formel IV, eingesetzt.
Ebenso zweckmässig kann die Umsetzung in der zweiten Stufe nach Bildung des Zwischenproduktes der allgemeinen Formel VI in Gegenwart einer Base durchgeführt werden Als Base können Alkalimetallalkoholate, wie Natriummethylat oder Natriumethylat, und Alkalimetallhydroxide, wie Natrium- oder Kaliumhydroxid, jeweils gegebenenfalls im entsprechenden Alkohol bzw. in Wasser gelöst, eingesetzt werden.

Nach einer üblichen Umsetzungszeit von insgesamt 5 bis 15 Stunden erhält man die Verbindung der allgemeinen Formel I welche nach üblichen Methoden aufgearbeitet werden kann.

Iminokohlensäurediester, welche als Ausgangsmaterial für die erfindungsgemässe Herstellung der Alkoxytriazolinone eingesetzt werden, können alternativ zu den in EP-A 0 523 619 und Hantzsch (Chem. Ber., 1895, 28, 2470 - 2471) beschriebenen Verfahren hergestellt werden.

In einer ersten Stufe wird hierbei ein Alkohol der allgemeinen Formel

R¹―OH VII

worin R¹ die genannte Bedeutung hat, mit einem Alkalimetall zum entsprechenden Alkoholat umgesetzt.
Als Alkohol können geradkettige oder verzweigte Alkohole mit 1 bis 6 C-Atomen eingesetzt werden. Namentlich erwähnt seien Methanol, Ethanol, Propanol, Isopropanol, Butanol, Isobutanol, tert-Butanol, Pentanol und seine Isomeren sowie Hexanol und seine Isomeren. Bevorzugt wird Methanol eingesetzt.
Als Alkalimetall kann Natrium oder Kalium eingesetzt werden Bevorzugt wird Natrium eingesetzt.
Die Umsetzung in der ersten Stufe erfolgt zweckmässig bei einer Temperatur von 10 bis 80 °C, vorteilhaft bei 20 bis 40 °C.

In der zweiten Stufe wird das Alkoholat mit einem Halogencyan zum Endprodukt der Formel II umgesetzt.
Als Halogencyan wird Chlorcyan oder Bromcyan, bevorzugt Chlorcyan eingesetzt. Die Umsetzung in der zweiten Stufe erfolgt zweckmässig bei einer Temperatur von -20 bis 20 °C, vorteilhaft bei -10 bis 5 °C.
Nach einer üblichen Umsetzungszeit von 1 bis 2 Stunden kann die Verbindung der allgemeinen Formel II isoliert werden oder direkt, ohne Isolation, als Ausgangsmaterial für die erfindungsgemässe Herstellung der Alkoxytriazolinone eingesetzt werden.
Vorzugsweise wird die Verbindung der allgemeinen Formel II direkt für die erfindungsgemässe Herstellung der Alkoxytriazolinone eingesetzt.

### Beispiele

### Beispiel 1:

### Herstellung von N'-Dimethoxymethylen-hydrazincarbonsäure-methylester

27,9 g (0,30 mol) Carbazinsäuremethylester wurden in Wasser (50 ml) vorgelegt und unter Kühlung mit 28,9 g (0,30 mol) HCl (37,9 %ig) versetzt. Bei 0 - 7 °C wurde eine Lösung von 27,2 g (0,30 mol) Iminokohlensäure-dimethylester in Wasser zugegeben. Anschliessend liess man noch 1 Stunde und 40 min bei 0 °C rühren. Das Reaktionsgemisch wurde mit Essigester extrahiert, die organische Phase über Magnesiumsulfat getrocknet und anschliessend im Vakuum eingeengt. Man erhielt 37,9 g (77,8 %) *N*'-Dimethoxymethylen-hydrazincarbonsäuremethylester in Form eines farblosen Feststoffs, der aus Toluol umkristallisiert wurde.
Schmelzpunkt 78,5 - 81,5 °C.

| | |
|---|---|
| ¹H-NMR (DMSO-d⁶, 400 MHz): δ = | 3,55 (s, 3H); |
| | 3,73 (s, 3H); |
| | 3,74 (s, 3H); |
| | 8,86 (1H). |

### Beispiel 2:

### Herstellung von N'-Dimethoxymethylen-hydrazincarbonsäure-methylester

10,6 g (0,263 mol) NaOH (fest) wurden in Methanol (64,1 g; 2,00 mol) gelöst. Anschliessend wurde auf 4 °C abgekühlt und mit der Einleitung von 15,5 g (0,25 mol) Chlorcyan begonnen. Man leitete insgesamt 1 Stunde ein und liess die Suspension von Iminokohlensäure-dimethylester 1 Stunde bei 5 °C nachrühren (Herstellung des Iminokohlensäure-dimethylesters in Anlehnung an EP-A 0 523 619).
23,2 g (0,25 mol) Carbazinsäuremethylester wurden in Wasser (100 ml) gelöst. Zu dieser Lösung wurde bei 4 - 6 °C die erhaltene Suspension von Iminokohlensäure-dimethylester während 30 min zugegeben. Durch gleichzeitige Zugabe von HCI (15 %ig; 48,8 ml) wurde der pH konstant bei 7 gehalten. Nach erfolgter Zugabe liess man noch 1 Stunde bei 3 °C rühren und engte die Lösung im Vakuum auf 110 g ein. Das Reaktionsgemisch wurde mit Essigester extrahiert, die organische Phase über Magnesiumsulfat getrocknet und anschliessend im Vakuum eingeengt. Man erhielt 25,3 g (52,9 %; Gehalt 85 %) *N*'-Dimethoxymethylenhydrazincarbonsäure-methylester in Form eines farblosen Feststoffs, der aus Toluol umkristallisiert wurde.
Schmelzpunkt 78,5 - 81,5 °C.

### Beispiel 3:

### a) Herstellung von Iminokohlensäure-dimethylester

64,1 g (2,00 mol) Methanol wurden mit 6,16 g (0,268 mol) Natriummetall versetzt Nachdem alles Natrium gelöst war, wurde auf 3 °C abgekühlt und begonnen, 15,5 g (0,25 mol) Chlorcyan einzuleiten. Man leitete insgesamt 1 Stunde und 15 min ein und liess die Suspension von Iminokohlensäure-dimethylester 1 Stunde bei 0 °C nachrühren.

### b) Herstellung von N'-Dimethoxymethylen-hydrazincarbonsäure-methylester

23,2 g (0,25 mol) Carbazinsäuremethylester wurden in Wasser (100 ml) gelöst. Zu dieser Lösung wurde bei 5 - 10 °C die in Beispiel 3a) erhaltene Suspension von Iminokohlensäure-dimethylester während 30 min zugegeben. Durch gleichzeitige Zugabe von HCl (15 %ig; 52,1 ml) wurde der pH konstant bei 7 gehalten. Nach erfolgter Zugabe liess man noch 1 Stunde 30 min bei 3 °C rühren und engte die Lösung im Vakuum auf 110 g ein. Das Reaktionsgemisch wurde mit Essigester extrahiert, die organische Phase über Magnesiumsulfat getrocknet und anschliessend im Vakuum eingeengt. Man erhielt 20,8 g (47,2 %; Gehalt 92 %) *N*'-Dimethoxymethylen-hydrazincarbonsäure-methylester in Form eines farblosen Feststoffs, der aus Toluol umkristallisiert wurde.
Schmelzpunkt 78,5 - 81,5 °C.

### Beispiel 4:

### Herstellung von N'-Dipropoxymethylen-hydrazincarbonsäure-methylester

396,4 g (9,812 mol) NaOH (fest) wurden in Propanol (3600 g; 59,3 mol) gelöst. Anschliessend wurde auf 0 bis 5°C abgekühlt und mit der Einleitung von 586,2 g (9,345 mol) Chlorcyan begonnen. Man leitete insgesamt 3 Stunden ein und erhielt eine hellgelbe, feine Suspension von Iminokohlensäure-dipropylester.
772,1 g (8,4 mol) Carbazinsäuremethylester wurden in Wasser (602 ml) gelöst. Zu dieser Lösung wurde bei Raumtemperatur die erhaltene Suspension von Iminokohlensäuredipropylester während 1 Stunde zugegeben. Durch gleichzeitige Zugabe von HCl (6 %ig) wurde der pH konstant bei 6,8 bis 7 gehalten. Nach erfolgter Zugabe wurde mit Propanol und Wasser nachgespült und man liess noch 2 Stunden rühren. Das Reaktionsgemisch wurde mit Essigester extrahiert, aus Hexan umkristallisiert und anschliessend im Vakuum getrocknet. Man erhielt 1391 g (71,6%; Gehalt 94,4%) N'-Dipropoxymethylenhydrazincarbonsäuremethylester in Form eines farblosen Feststoffs.

Schmelzpunkt: 38,1 - 38,3 °C

| | |
|---|---|
| ¹H-NMR-Daten (CDCl₃, 400 MHz): δ = | 0,97 (t, 6H); |
| | 1,77 (m, 4H); |
| | 3,76 (s, 3H); |
| | 4,08 (t, 2H); |
| | 4,15 (mₙ 2H); |
| | 7,28 (s, NH). |

### Beispiel 5:

### Herstellung von N'-Diphenoxymethylen-hydrazincarbonsäure-methylester

4,31 g (46,9 mmol) Carbazinsäuremethylester wurden in Wasser (40 ml) vorgelegt und mit 4,75 g (46,9 mmol) konz. Salzsäure (36 %ig) versetzt. 10,0 g (46,9 mmol) Diphenylimidocarbonat (Herstellung des Diphenylimidocarbonats in Anlehnung an Heydayatullah, M. *Bull Soc. Chim Fr*. **1967**, 416) wurden bei 7 °C zu dieser Lösung portionsweise zugegeben. Nach 4 Stunden Rühren unter Eisbadkühlung wurde mit Essigester (4 x 100 ml) extrahiert. Die vereinten organischen Phasen wurden über Magnesiumsulfat getrocknet und nach Filtration im Vakuum eingeengt. Der Rückstand wurde im Vakuum bei 25 °C getrocknet. Man erhielt 7,62 g Rohprodukt, das Phenol und N'-Diphenoxymethylenhydrazincarbonsäure-methylester im Verhältnis 10 : 1 enthielt. Die Ausbeute an N'-Diphenoxymethylen-hydrazincarbonsäure-methylester betrug 693 mg (5 %).

| | |
|---|---|
| ¹H-NMR (DMSO-d⁶, 400 MHz): δ = | 3,59 (s, 3H); |
| | 7,21 -7,50 (m, 10H); |
| | 9.05 (s, 1H). |

### Beispiel 6:

### Herstellung von N'-(Methoxymethylaminomethylen)hydrazincarbonsäuremethylester

0,95 g (5,00 mmol) N'-Dimethoxymethylen-hydrazincarbonsäure-methylester (85 %ig) wurden mit 3,57 g einer 44 %igen Lösung von Methylamin (50 mmol) in Methanol versetzt und 3 Tage bei Raumtemperatur gerührt. Nach Aufarbeitung und Entfernung des Lösungsmittels wurden 0,85 g (76 %) N'-(Methoxymethylaminomethylen)-hydrazincarbonsäure-methylester in Form eines Öls erhalten.

| | |
|---|---|
| ¹H-NMR (DMSO-d⁶, 400 MHz): δ = | 2,58 (d, 3H); |
| | 3,54 (s, 3H); |
| | 3,65 (s, 3H); |
| | 5,98 (s, 1H); |
| | 8,33 (s, 1H). |

### Beispiel 7:

### Herstellung von 5-Methoxy-4-methyl-2,4-dihydro-1,2,4-triazol-3-on

4,77 g (25,0 mmol) N'-Dimethoxymethylen-hydrazincarbonsäure-methylester (85 %ig) wurden mit 15,3 g einer 51 %igen Lösung von Methylamin (250 mmol) in Methanol versetzt und 3 Tage bei Raumtemperatur gerührt. Die orangerote Lösung wurde im Vakuum eingeengt um überschüssiges Methylamin zu entfernen. Man erhielt den rohen N'-(Methoxymethylaminomethylen)hydrazincarbonsäuremethylester, der anschliessend in Methanol (15 ml) aufgenommen und mit 4,95 g (27,5 mmol) Natriummethylat-Lösung (30 % in Methanol) auf 50 °C erhitzt wurde. Nach 3 h 50 min wurde das Reaktionsgemisch auf Raumtemperatur abgekühlt, mit 2N HCI neutralisiert und im Vakuum eingeengt. Der Rückstand wurde in Wasser (6 ml) aufgenommen und über Nacht im Kühlschrank aufbewahrt. Die farblosen Kristalle wurden abfiltriert, mit kaltem Wasser gewaschen und im Vakuum getrocknet. Man erhielt 1,70 g (52,7 %) 5-Methoxy-4-methyl-2,4-dihydro-1,2,4-triazol-3-on in Form eines farblosen Feststoffs. Schmelzpunkt 146 - 148 °C.

### Beispiel 8:

### Herstellung von 5-Methoxy-4-methyl-2,4-dihydro-1,2,4-triazol-3-on

8.11 g (0,05 mol) *N*'-Dimethoxymethylen-hydrazincarbonsäure-methylester wurden in 25,4 g Methanol gelöst und mit 3,26 g (0,105 mol) Methylamin, gelöst in Methanol, versetzt und auf 70 °C 14 Stunden im Autoklaven erhitzt. Das Reaktionsgemisch wurde im Vakuum eingeengt und in Wasser (10 ml) aufgenommen. Durch Zugabe von konz. HCl wurde angesäuert. Die bei 4 °C ausgefallenen Kristalle wurden abfiltriert und getrocknet. Man erhielt 1,89 g (29,3%) 5-Methoxy-4-methyl-2,4-dihydro-1,2,4-triazol-3-on in Form eines farblosen Feststoffs.
Schmelzpunkt 146 - 148 °C.

| | |
|---|---|
| ¹H-NMR (DMSO-d⁶, 400 MHz): δ = | 2,96 (s, 3H); |
| | 3,90 (s, 3H); |
| | 10.88 (s, 1H). |

### Beispiel 9:

### Herstellung von 5-Propoxy-4-methyl-2,4-dihydro-1,2,4-triazol-3-on

693,6 g (94,4 %) N'-Dipropoxymethylen-hydrazincarbonsäure-methylester (3 mol) wurden in 1752 g Methanol gelöst und mit 564,7 g (6 mol) Methylamin, gelöst in Ethanol, versetzt und auf 100 °C 20 Stunden im Autoklaven erhitzt. Das Reaktionsgemisch wurde im Vakuum eingeengt, in Wasser (880 ml) aufgenommen, mit Methylenchlorid extrahiert und im Vakuum eingeengt. Die beim Abkühlen ausgefallenen Kristalle wurden abfiltriert und getrocknet. Man erhielt 383 g (71,2 %; Gehalt 87,6%) 5-Propoxy-4-methyl-2,4-dihydro-1,2,4-triazol-3-on in Form eines Feststoffs.

Schmelzpunkt: 72,5 - 73,5 °C

| | |
|---|---|
| ¹H-NMR-Daten (DMSO-d⁶, 400 MHz): δ = | 0,96 (t, 3H); |
| | 1,74 (m, 2H); |
| | 2,99 (s, 3H); |
| | 4,17 (t, 2H); |
| | 10,86 (s, NH). |

### Beispiel 10:

### Herstellung von 5-Propoxy-4-methyl-2,4-dihydro-1,2,4-triazol-3-on

In einem Autoklaven wurden 10,02 g (44,3 mmol) N'-Dipropoxymethylenhydrazincarbonsäuremethylester, 3,94 g einer 39%igen Lösung von Methylamin (49,9 mmol) in Methanol und 46 g Methanol vorgelegt. Nach 3 Stunden bei 150 °C wurde das Lösungsmittel abdestilliert. Die Rohsubstanz wurde in Wasser (20 ml) aufgenommen und mit Methylenchlorid mehrmals extrahiert. Man engte die organische Lösung ein und erhielt 6,48 g 5-Propoxy-4-methyl-2,4-dihydro-1,2,4-triazol-3-on mit einem Gehalt von 50%, was einer Ausbeute von 46% entspricht.

### Beispiel 11:

### Herstellung von 4-Benzyl-5-propoxy-2,4-dihydro-1,2,4-triazol-3-on

In einem Autoklaven wurden 2,04 g N'-Dipropoxymethylenhydrazincarbonsäuremethylester (8,9 mmol) und 3,46 g Benzylamin (31,6 mmol) in 25,8 g Methanol vorgelegt. Nach 5 Stunden bei 110 °C wurde das Lösungsmittel abdestilliert und das 4-Methyl-5-benzyloxy-2,4-dihydro-1,2,4-triazol-3-an in Essigsäureethylester umkristallisiert. Man erhielt 0,57 g (27%) reines 4-Benzyl-5-propoxy-2,4-dihydro-1,2,4-triazol-3-on

| | |
|---|---|
| ¹H-NMR (DMSO-d⁶, 400 MHz): δ = | 10,96 (s, 1H); |
| | 7,2 - 7,4 (m, 5H); |
| | 4,62 (s, 2H); |
| | 4,14 (t, 2H; J = 6,3 Hz); |
| | 1,66 (m, 2H); |
| | 0,84 (t, 3H, J = 7,4 Hz). |

| | |
|---|---|
| ¹³C-NMR (DMSO-d⁶, 400 MHz) : δ = | 153,2 (s); |
| | 151,0 (s); |
| | 136,5 (s); |
| | 128,5 (d, 2C); |
| | 127,5 (d); |
| | 127,2 (d, 2C); |
| | 70,2 (t); |
| | 42,3 (t); |
| | 21,4 (t); |
| | 9,9 (q). |

### Beispiel 12:

### Herstellung von 4-Cyclohexyl-5-propoxy-2,4-dihydro-1,2,4-triazol-3-on

In einem Autoklaven wurden 2,04 g N'-Dipropoxymethylenhydrazincarbonsäuremethyester (8,9 mmol) und 3,23 g Cyclohexylamin (32,2 mmol) in 25,8 g Methanol vorgelegt. Nach 5 Stunden bei 110 °C wurde das Lösungsmittel abdestilliert und das rohe 4-Methyl-5-cyclohexyloxy-2,4-dihydro-1,2,4-triazol-3-on (1,3 g) durch eine Säulenchromatographie (Fliessmittel: Essigsaureethylester) gereinigt. Man erhielt 0,37 g (18%) reines Produkt.

Schmelzpunkt: 132 - 136 °C

| | |
|---|---|
| ¹H-NMR (DMSO-d⁶, 400 MHz). δ = | 10,81 (s, 1H); |
| | 4,15 (t, 2H, J = 6,3 Hz); |
| | 1,77 (m, 2H); |
| | 0,96 (t, 3H, J = 7,4 Hz); |
| | 3,71 (m, 1H); |
| | 1,0 - 2,0 (m). |

| | |
|---|---|
| ¹³C-NMR (DMSO-d⁶, 400 MHz): δ = | 152,8 (s); |
| | 151,2 (s); |
| | 70,1 (t); |
| | 51,0 (d); |
| | 29,9 (t, 2C); |
| | 25,2 (t, 2C); |
| | 24,7 (t); |
| | 21,5 (t); |
| | 10,1 (q). |

## Patentansprüche

1. Verfahren zur Herstellung von Alkoxytriazolinonen der allgemeinen Formel 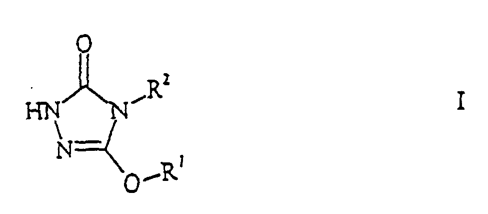 worin R¹ und R² unabhängig voneinander eine gegebenenfalls mit Halogen, Amino, Hydroxy, Alkylamino, Dialkylamino oder Alkoxy substituierte Alkylgruppe, Arylgruppe, Arylalkylgruppe oder Cycloalkylgruppe bedeuten, **dadurch gekennzeichnet, dass** in einer ersten Stufe ein Iminokohlensäurediester der allgemeinen Formel 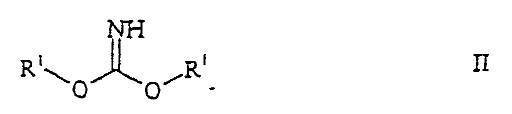 worin R¹ die genannte Bedeutung hat, in Gegenwart einer Mineralsäure und gelöst in Wasser oder in einem Gemisch aus Wasser mit einem mit Wasser mischbaren polaren organischen Lösungsmittel mit einem Carbazinsäureester der allgemeinen Formel 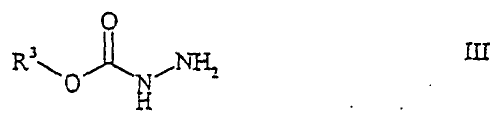 worin R³ eine gegebenenfalls wie oben substituierte Alkylgruppe bedeutet, zu einem Hydrazincarbonsäureester der allgemeinen Formel 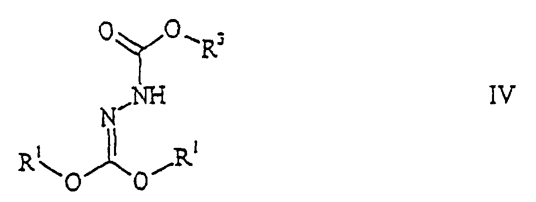 worin R¹ und R³ die genannte Bedeutung haben, umgesetzt wird, welcher in einer zweiten Stufe in Gegenwart von Wasser, einem polaren organischen Lösungsmittel oder Mischungen davon mit einem Amin der allgemeinen Formel
R²―NH₂ V
worin R² die genannte Bedeutung hat, zum Endprodukt der Formel I umgesetzt wird.

2. Verfahren nach Anspruch 1, **dadurch gekennzeichnet, daß** R¹ eine Methyl-, Propyl- oder Phenylgruppe ist.

3. Verfahren nach Anspruch 1 oder 2, **dadurch gekennzeichnet, dass** R² eine Methyl-, Benzyl- oder Cyclohexylgruppe ist.

4. Verfahren nach einem der Ansprüche 1 bis 3, **dadurch gekennzeichnet, dass** R³ eine Methylgruppe ist.

5. Verfahren nach einem der Ansprüche 1 bis 4, **dadurch gekennzeichnet, dass** die in der ersten Stufe eingesetzte Mineralsäure Salzsäure ist.

6. Verfahren nach einem der Ansprüche 1 bis 5, **dadurch gekennzeichnet, dass** die Umsetzung in der ersten Stufe bei einer Temperatur von -5 bis 40 °C durchgeführt wird.

7. Verfahren nach einem der Ansprüche 1 bis 6, **dadurch gekennzeichnet, dass** das in der zweiten Stufe eingesetzte polare organische Lösungsmittel ein geradkettiger oder verzweigter Alkohol mit 1 bis 6 C-Atomen ist.

8. Verfahren nach einem der Ansprüche 1 bis 7, **dadurch gekennzeichnet, dass** die Umsetzung in der zweiten Stufe bei einer Temperatur von 10 bis 200 °C durchgeführt wird.

9. Verfahren nach einem der Ansprüche 1 bis 8, **dadurch gekennzeichnet, dass** das Zwischenprodukt der Formel IV isoliert wird.

10. Hydrazincarbonsäureester der allgemeinen Formel 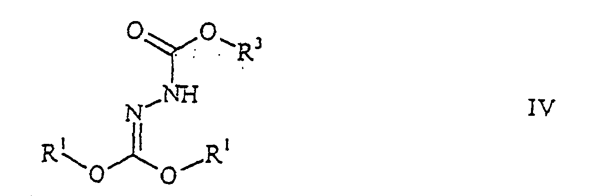 worin R¹ eine gegebenenfalls mit Halogen, Amino, Hydroxy, C₁₋₆-Alkylamino, Di-C₁₋₆-alkylamino oder C₁₋₆-Alkoxy substituierte geradkettige oder verzweigte C₁₋₆-Alkylgruppe, Phenyl, Phenyl-C₁₋₆-alkyl oder C₃₋₆-Cycloalkyl und R³ eine gegebenenfalls mit Halogen, Amino, Hydroxy, C₁₋₆-Alkylamino, Di-C₁₋₆-alkylamino oder C₁₋₆-Alkoxy substituierte geradkettige oder verzweigte C₁₋₆-Alkylgruppe bedeuten, mit der Einschränkung, daß R¹ nicht Methyl ist, wenn R³ Ethyl ist.

11. Hydrazincarbonsäure nach Anspruch 10, nämlich N'-Dimethoxymethylen-hydrazincarbonsäure-methylester.

12. Hydrazincarbonsäure nach Anspruch 10, nämlich N'-Dipropoxymethylen-hydrazincarbonsäure-methylester.

13. Hydrazincarbonsäure nach Anspruch 10, nämlich N'-Diphenoxymethylen-hydrazincarbonsäure-methylester.

## Claims

1. Process for preparing alkoxytriazolinones of the general formula 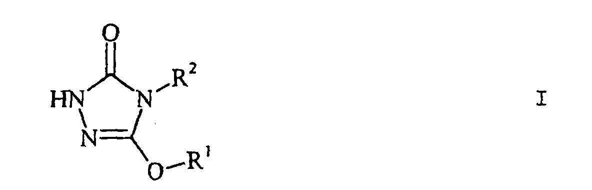 in which R¹ and R² independently of one another are an alkyl group, aryl group, arylalkyl group or cycloalkyl group optionally substituted with halogen, amino, hydroxy, alkylamino, dialkylamino or alkoxy, **characterized in that**, in a first step, an iminocarbonic acid diester of the general formula 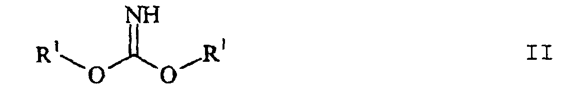 in which R¹ has the above mentioned meaning, is reacted, in the presence of a mineral acid and dissolved in water or in a mixture of water with a water-miscible polar organic solvent, with a carbazinic acid ester of the general formula 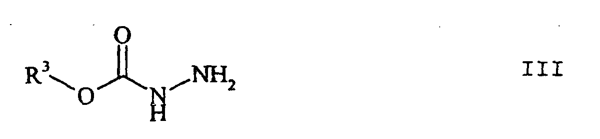 in which R³ is an alkyl group optionally substituted as above to give a hydrazinecarboxylic acid ester of the general formula 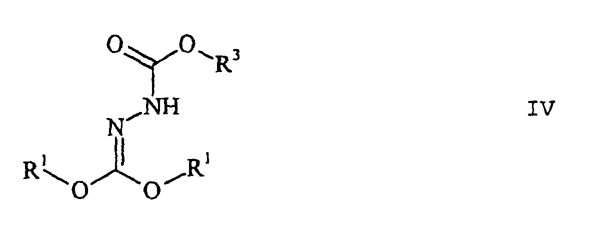 in which R¹ and R³ have the above mentioned meaning, which is in a second step reacted in the presence of water, a polar organic solvent or mixtures thereof, with an amine of the general formula
R²―NH₂ V
in which R² has the meaning mentioned, to give the end product of the formula I.

2. Process according to claim 1, **characterized in that** R¹ is a methyl, propyl, or phenyl group.

3. Process according to claim 1 or 2, **characterized in that** R² is a methyl, benzyl or cyclohexyl group.

4. Process according to any one of claims 1 to 3, **characterized in that** R³ is a methyl group.

5. Process according to any one of claims 1 to 4, **characterized in that** the mineral acid used in the first step is hydrochloric acid.

6. Process according to any one of claims 1 to 5, **characterized in that** the reaction of the first step is carried out at a temperature from -5 to 40°C.

7. Process according to any one of claims 1 to 6, **characterized in that** the polar organic solvent used in the second step is a straight-chain or branched alcohol having 1 to 6 C atoms.

8. Process according to any one of claims 1 to 7, **characterized in that** the reaction of the second step is carried out at a temperature from 10 to 200°C.

9. Process according to any one of claims 1 to 8, **characterized in that** the intermediate of formula IV is isolated.

10. Hydrazinecarboxylic acid ester of the general formula 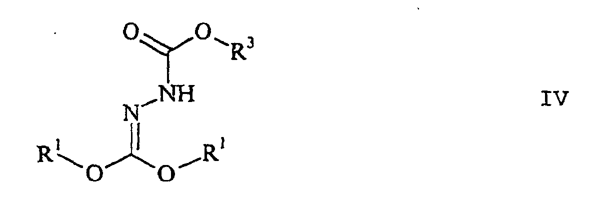 in which R¹ is a straight-chain or branched C₁₋₆ alkyl group optionally substituted with halogen, amino, hydroxy, C₁₋₆-alkylamino, di-C₁₋₆-alkylamino; phenyl, phenyl-C₁₋₆-alkyl or C₃₋₆-cycloalkyl, and R³ is a straight-chain or branched C₁₋₆ alkyl group optionally substituted with halogen, amino, hydroxy, C₁₋₆-alkylamino, di-C₁₋₆-alkylamino or alkoxy, with the proviso that R¹ is not methyl if R³ is ethyl.

11. Hydrazinecarboxylic acid ester according to claim 10, namely, methyl N'-dimethoxymethylenehydrazine carboxylate.

12. Hydrazinecarboxylic acid ester according to claim 10, namely, methyl N'-dipropoxymethylenehydrazine carboxylate.

13. Hydrazinecarboxylic acid ester according to claim 10, namely, methyl N'-diphenoxymethylenehydrazine carboxylate.

## Revendications

1. Procédé de préparation d'alcoxytriazolinones de formule générale : 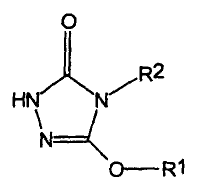 où R¹ et R² représentent indépendamment l'un de l'autre, un radical alkyle, un radical aryle, un radical arylalkyle où un radical cycloalkyle, le cas échéant substitué par un halogène, un radical amino, hydroxy, alkylamino, dialkylamino ou alcoxy, **caractérisé en ce que**, dans une première étape, on transforme un diester d'acide iminocarboxyl de formule générale : 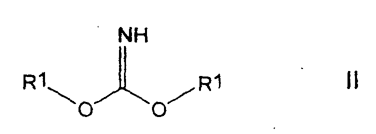 où R¹ a la signification indiquée par réaction, en présence d'un acide minéral et dissous dans l'eau ou dans un mélange d'eau avec un solvant organique polaire miscible avec l'eau, avec un ester d'acide carbazique de formule générale 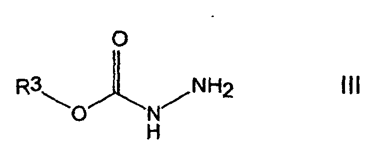 où R³ représente un radical alkyle le cas échéant substitué comme ci-dessus, en un ester d'acide hydrazinecarboxylique de la formule générale : 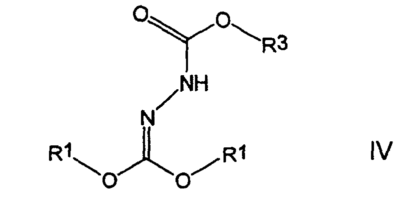 où R¹ et R³ ont la signification indiquée, lequel est transformé dans une deuxième étape, en présence d'eau, d'un solvant organique polaire ou d'un mélange de ceux-ci, par réaction avec une aminé de formule générale :
R² - NH₂ V
où R² a la signification indiquée, en le produit final de formule I.

2. Procédé selon la revendication 1, **caractérisé en ce que** R¹ est un radical méthyle, propyle ou phényle.

3. Procédé selon la revendication 1 ou 2, **caractérisé en ce que** R² est un radical méthyle, benzyle ou cyclohexyle.

4. Procédé selon l'une des revendications 1 à 3, **caractérisé en ce que** R³ est le radical méthyle.

5. Procédé selon l'une des revendications 1 à 4, **caractérisé en ce que** l'acide minéral mis en oeuvre dans la première étape est l'acide chlorhydrique.

6. Procédé selon l'une des revendications 1 à 5, **caractérisé en ce que** la réaction est réalisée dans la première étape, à une température de -5 à 40°C.

7. Procédé selon l'une des revendications 1 à 6, **caractérisé en ce que** le solvant organique polaire mis en oeuvre dans la deuxième étape est un alcool rectiligne ou ramifié ayant de 1 à 6 atomes C.

8. Procédé selon l'une des revendications 1 à 7, **caractérisé en ce que** la réaction est réalisée dans la deuxiême étape, à une température allant de 10 à 200°C.

9. Procédé selon l'une des revendications 1 à 8, **caractérisé en ce que** le produit intermédiaire de formule IV est isolé.

10. Ester d'acide hydrazinecarboxylique de formule générale : 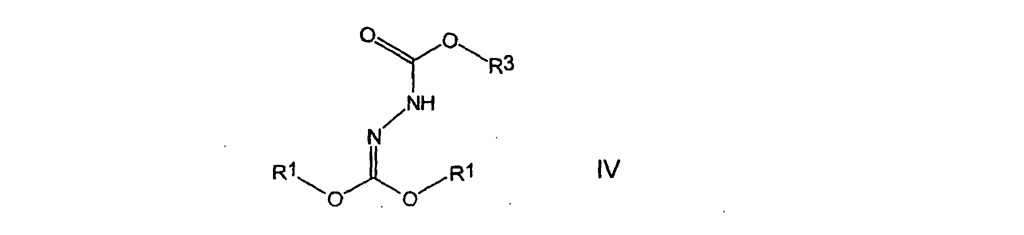 où R¹ représente un radical alkyle rectiligne ou ramifié en C₁-C₆, un radical phényle, un radical phénylalkyle rectiligne ou ramifié en C₁-C₆ ou un radical cycloalkyle rectiligne ou ramifié en C₃-C₆, le cas échéant substitué par un halogène, un radical amino, hydroxyle, (alkyl en C₁-C₆)amino, di(alkyl en C₁-C₆)amino ou alcoxy en C₁-C₆, et R³ représente un radical alkyle en C₁-C₆ linéaire ou ramifié, le cas échéant substitué par un halogène, un radical amino, hydroxy, (alkyl en C₁-C₆)amino, di(alkyl en C₁-C₆)amino ou alcoxy en C₁-C₆, avec la limitation que R¹ n'est pas méthyle lorsque R³ est éthyle.

11. Ester d'acide hydrazinecarboxylique selon la revendication 10, à savoir l'ester méthylique de l'acide N'-diméthoxyméthylènehydrazinecarboxylique.

12. Ester d'acide hydrazinecarboxylique selon la revendication 10, à savoir l'ester méthylique de l'acide N'-dipropoxyméthylènehydrazinecarboxylique.

13. Ester d'acide hydrazinecarboxylique selon la revendication 10, à savoir l'ester méthylique de l'acide N'-diphénoxyméthylènehydrazinecarboxylique.
